# EUROPEAN PATENT SPECIFICATION

(11) **EP 3 269 315 B1**
(45) Date of publication and mention of the grant of the patent: **02.03.2022**
(21) Application number: 17181870.1
(22) Date of filing: 15.08.2013
(51) Int. Cl.: A61B 17/22, A61B 17/00

(54) **ELECTROHYDRAULIC LITHOTRIPSY PROBE AND ELECTRICAL SOURCE FOR AN ELECTROHYDRAULIC LITHOTRIPSY PROBE**
ELEKTROHYDRAULISCHE LITHOTRIPSIESONDE UND STROMQUELLE FÜR DIE ELEKTROHYDRAULISCHE LITHOTRIPSIESONDE
SONDE DE LITHOTRIPSIE ÉLECTROHYDRAULIQUE ET SOURCE ÉLECTRIQUE POUR SONDE DE LITHOTRIPSIE ÉLECTROHYDRAULIQUE

(30) Priority: 17.08.2012 US 201261684353 P; 13.03.2013 US 201313800686
(43) Date of publication of application: 17.01.2018
(62) Divisional of application: 13779341.0
(73) Proprietor: Northgate Technologies Inc., Elgin, IL 60123 (US)
(72) Inventor: Mantell, Robert, Arlington Heights, IL 60004 (US); Curtis, Chip, West Dundee, IL 60118 (US); Sobelevsky, Mikhail, Streamwood, IL 60107 (US)
(74) Representative: Grünecker Patent- und Rechtsanwälte PartG mbB

(56) References cited:
- DE-A1- 3 038 445
- US-A- 4 030 505
- US-A- 4 691 706
- US-A- 4 721 107
- US-A- 4 905 673
- US-A1- 2004 082 859
- US-A1- 2009 204 134
- US-A1- 2009 213 504
- US-A1- 2010 016 862
- US-A1- 2010 036 294

## Description

### BACKGROUND

Electrohydraulic lithotripsy has been used in the medical field, primarily for breaking concretions in the urinary or biliary track. Conventional lithotripsy probes produce a shockwave that radiates axially from a distal end of the lithotripsy probe. While a shockwave radiating axially from a distal end of a lithotripsy probe is useful in breaking up concretions such as a kidney stone, it is often difficult to use these types of probes to break up annular concretions, such as concretions around a heart valve. Accordingly, improved lithotripsy probes are desirable for breaking up concretions that are not located axially from a distal end of a lithotripsy probe.

Additionally, traditional electrohydraulic lithotripsy systems are powered by electrical sources that operate in one of two manners. In a first method, an electrical source includes two electrical contacts separated by an air gap. During operation a high voltage builds up on one of the electrical contacts until an electrical arc occurs over the air gap between the two electrical contacts. The electrical arc results in the electrical source sending a voltage and a current to a lithotripsy probe, where the voltage and current cause a shockwave. Because the air gap within the electrical source provides the dielectric barrier for the electric arc, it is often difficult to precisely control the voltage and current sent to the lithotripsy probe from day to day, or from spark to spark, due to environmental conditions such as temperature, humidity, and/or dust. The inconsistencies in the voltage and current sent to the lithotripsy probe result in the lithotripsy probe producing inconsistent shockwaves.

In a second method, an electrical source includes three electrodes positioned within a large ceramic tube that is infused with ionized gas. When a voltage is applied to the ceramic tube, the gas within the ceramic tube begins to ionize, thereby allowing electricity to flow from one internal electrode to another. The electric arc between the electrodes in the ceramic tube results in the electrical source sending a voltage and a current to a lithotripsy probe, where the voltage and current cause a shockwave. The large ceramic tubes infused with ionized gas can be expensive and require large transformers to operate.

DE 3038445 A1 discloses a pressure wave generator for medical applications comprising a catheter having a tip with a discharge electrode surrounded by a ballon. The ballon can be filled with a fluid by means of a pump extracting the fluid from a pressurized supply.

US 4691706 A shows a power supply for a calculus disintegrating apparatus comprising an electrical circuit with discharge tubes connected to a capacitor.

Improved electrical sources are desirable that are able to cause a lithotripter probe to create consistent shockwaves without large expensive ceramic tubes that are ionized with gas.

The present invention provides a an invasive lithotripter probe system having the features as set out in claim 1.

### BRIEF DESCRIPTION OF THE DRAWINGS

FIG. 1 is a perspective view of an electrohydraulic lithotripsy probe;
FIG. 2 is a cross-sectional side view of the electrohydraulic lithotripsy probe of FIG. 1;
FIG. 3 is a cross-sectional view of the electrohydraulic lithotripsy probe of FIG. 1 with channels for infusing a liquid in a flexible encapsulating member of the probe and extracting a liquid from the flexible encapsulating member of the probe;
FIG. 4a is a circuit diagram of one implementation of a power generator for an intracorporeal electrohydraulic lithotripter;
FIG. 4b is an illustration of an example high voltage pulse generated by the power generator of FIG. 4a;
FIG. 5 is a circuit diagram of another implementation of a power generator for an intracorporeal electrohydraulic lithotripter;
FIG. 6a is a cross-section of an electrohydraulic lithotripsy probe including a fusible link;
FIG. 6b is a flow chart for a method of using the electrohydraulic lithotripsy probe of FIG. 6a;
FIG. 7a is a cross-section of an electrohydraulic lithotripsy probe including a smart memory chip;
FIG. 7b is a flow chart of a method for using the electrohydraulic lithotripsy probe of FIG. 7b;
FIG. 8a is a cross-section of an electrohydraulic lithotripsy probe including a fusible link and a smart memory chip;
FIG. 8b is a flow chart of a method for using the electrohydraulic lithotripsy probe of FIG. 8a;
FIG. 9a is a cross-section of an electrohydraulic lithotripsy probe including a first fusible link and a second fusible link; and
FIG. 9b is a flow chart of a method for using the electrohydraulic lithotripsy probe of FIG. 9a.

### DETAILED DESCRIPTION OF THE DRAWINGS

The present disclosure is directed to invasive electrohydraulic lithotripsy probes that creates a shockwave for uses such as breaking up concretions that are at least semi-annular or disrupting tissue of a body organ. In some implementations, the shockwave is substantially spherical in shape at first, and then changes to a substantially toroid shape (also known as doughnut shaped). However, in other implementations the shockwave may be substantially spherical in shape or substantially toroid in shape. The present disclosure is additionally directed to electrical sources that utilizes multiple spark gaps to provide voltage and current to an invasive electrohydraulic lithotripsy probe in a way that results in consistent shockwave generation.

Inventive embodiments of the disclosed electrohydraulic lithotripsy ("EHL") probes include a first conic electrode at a distal end of the probe, and a second conic electrode coaxially aligned with the first electrode. A difference in voltage polarities between the first and second conic electrodes causes an electric arc, resulting in a shockwave that radiates from the lithotripsy probe, where the shockwave is substantially spherical in shape at first and then changes to a substantially toroid shape.

In one implementation, the EHL probes described below may be delivered to a proper channel of a heart by threading (or pre-loading) an EHL probe through a center lumen of a catheter or balloon device. The catheter may be threaded through appropriate veins or arteries to address concretions either forming in vessels or even in the valves of the heart or other organs.

In other implementations, the EHL probes described below may be delivered to a small lumen of a body organ for the purpose of disturbing or disrupting (distressing) tissue of the body organ in such a way as to cause a stricture or a "scarring" of the tissue for the purpose of creating a permanent stricture or blockage of the lumen.

For example, the EHL probes described below may be used to purposely create a blockage in a fallopian tube for the purpose of preventing pregnancies (sterilization). A fallopian tube, which is approximately 1 mm in diameter, would have one of the EHL probes described below threaded into it. The EHL probe may be threaded through the fallopian tube(s) to address the inner surface of the fallopian tube. Upon generating the EHL spark and subsequent pressure wave, a rupturing or disrupting of the walls of the inner surface of the fallopian tube can be accomplished. The subsequent healing or scarring of the walls cause the walls of the inner surface of the fallopian tube to knit together and create a blockage that renders the fallopian tube non-functional, thereby sterilizing a patient and preventing pregnancies.

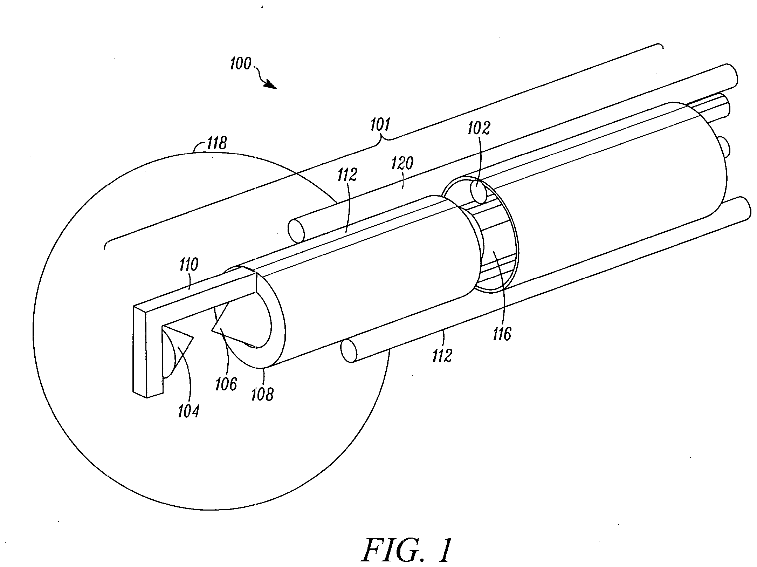

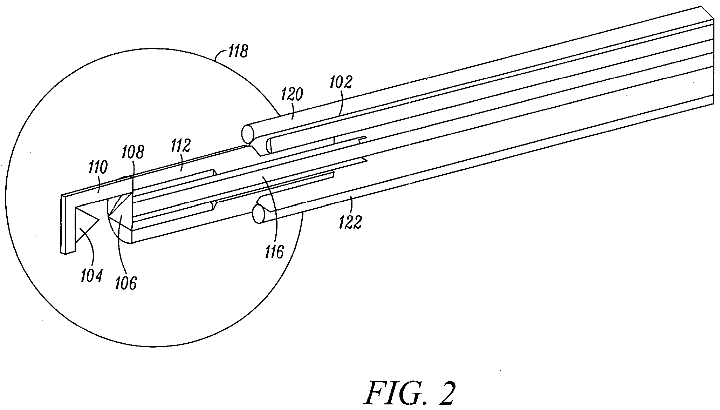

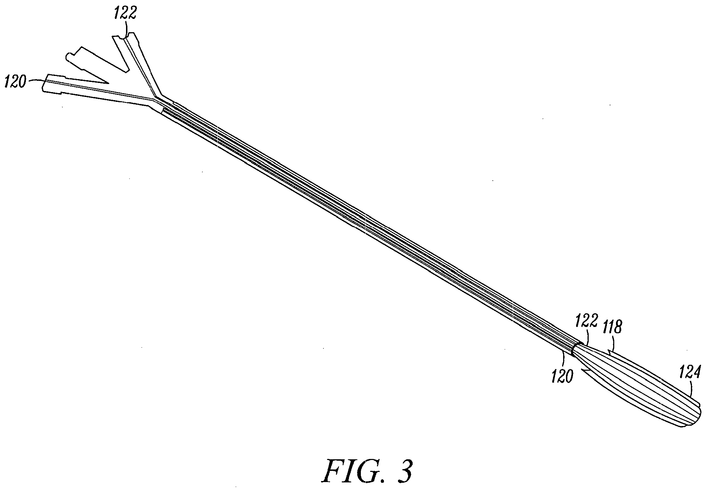

Referring to FIGS. 1-3, an EHL probe 100 (the "probe 100") includes a lithotripsy probe tip 101 including an insulating body 102, a first conic electrode 104, and a second conic electrode 106. Typically, the first conic electrode 104 is positioned at a first distal end 108 of the lithotripsy probe tip 101. In some implementations, the first conic electrode 104 includes an electrically conductive material such as copper, silver, or stainless steel.

Additionally, in some implementations not forming part of the claimed invention , the first electrode 104 may be shapes other than conic, such as a curved surface.

The first conic electrode 104 is supported by a structure 110 extending from the first distal end 108 of the lithotripsy probe tip 101. The support structure 110 may include an electrically conductive material, such as copper, silver, stainless steel, or other conductive materials, and electrically coupled with a first electrically conductive structure 112 in the EHL probe 100. Typically, the support structure 110 is insulated other than where the support structure 110 is electrically coupled with the first conic electrode 104 and the first electrically conductive structure 112. As known in the art, the first conductive structure 112 may be coupled with an electrical source, such as the electrohydraulic generator described below, to charge the first conic electrode 104 to a first polarity.

The second conic electrode 106 is positioned in the body of the lithotripsy probe tip 101. In some implementations, the second conic electrode 106 includes an electrically conductive material such as copper, silver, stainless steel, or other conductive materials.

Additionally, in some implementations not forming part of the claimed invention , the second electrode 106 may be shapes other than conic, such as a curved surface.

The second conic electrode 106 is positioned in the lithotripsy probe tip 101 such that the second conic electrode 106 is coaxially, and in some implementations symmetrically, aligned with the first conic electrode 104. For example, the first and second conic electrodes 104, 106 may be positioned such that an axis extending from the first conic electrode 104 Is substantially aligned with an axis extending from the second conic electrode 106.

In some implementations, a distance between a tip of the first conic electrode 104 and a tip of the second conic electrode 106 is approximately 0.5334 mm ( 0.021 inch ) . However, various distances between approximately 0.1524 mm and 2.54 mm ( 0.006 and 0.100 inch ) could be used between the tips of the electrodes depending on the application and the amount of energy to be transmitted.

The second conic electrode 106 is electrically coupled with a second electrically conductive structure 116 in the EHL probe 100. As known in the art, the second electrically conductive structure 116 may be coupled with an electrical source and used to charge the second conic electrode 106 to a second polarity, which is opposite to the first polarity of the first conic electrode 104.

In one implementation, the first conic electrode 104 is an anode and the second conic electrode 106 is a cathode, where in other implementations, the first conic electrode 104 is a cathode and the second conic electrode 106 is an anode. When the first conic electrode 104 is charged to a first polarity via the first conductive structure 112 and the second conic electrode 106 is charged to a second, opposite polarity via the second conductive structure 114, a discharge of electricity occurs between the first and second conic electrodes 104, 106 (an electrical arc) when the potential between the first and second conic electrodes 104, 106 reaches the breakdown voltage for the media separating the electrodes.

According to the invention, directed to implementations, such as the heart application described above, at least a portion of the lithotripsy probe tip 101 including the first and second conic electrodes 104, 106 is surrounded by a flexible encapsulating member 118, such as a balloon, comprising a water-tight flexible material such as Mylar. The flexible encapsulating member 118 encapsulates a liquid such as saline. However, other liquids can be used.

When an electrical arc occurs between the first and second conic electrodes 104, 106 as described above, the electrical arc causes a steam bubble in the liquid of the flexible encapsulating member 118. The steam bubble rapidly expands and contracts back on itself. As the steam bubble contracts, a pressure wave (a shockwave) is created in the liquid of the flexible encapsulating member 118 that radiates away from the lithotripsy tip 101 in a substantially toroid shape such that the shockwave is at least semi-annular. In some implementations, the electrical arc causes a shockwave that is substantially spherical in shape at first, and then changes to a substantially toroid shape. Additionally, in some implementations not forming part of the claimed invention , a flexible encapsulating member 118 does not surround the lithotripsy probe tip 101.

The EHL probe 100 includes a first channel (or lumen) 120 and may further include a second channel (or lumen) 122 that are each in communication with an interior of the flexible encapsulating member 118. During operation, the first channel 120 is utilized to infuse a liquid, such as saline, into an interior of the flexible encapsulating member 118 for the purpose of expanding the flexible encapsulating member 118 and providing a medium for creating electrohydraulic effect.

Additionally, the second channel 122 may be utilized to remove the liquid from the interior of the flexible encapsulating member 118 and collapse the flexible encapsulating member 118. In some implementations, the second channel 122 may further be utilized to degass the fluid within the flexible encapsulating member 118 after an electrohydraulic discharge between the first and second conic electrodes 104, 106.

The circulation of fluid through the interior of the flexible encapsulating member 118 using the first and second channels 120, 122 may be done through manual means such as a syringe, mechanical means such as a pump, or any other means known in the art.

According to the invention, at least the the first channel, and, preferabably also the second channels 120, 122 may-include one or more valves, membranes, or cartridges to assist in injecting a fluid into the interior region of the flexible encapsulating member 118, removing a fluid from the interior region of the flexible encapsulating member 118, or degassing the fluid within the interior region of the flexible encapsulating member 118.

A valve or membrane positioned in or adjacent to the first channel 120 may allows a fluid to flow into the interior region of the flexible encapsulating member 118 while preventing the fluid from entering the first channel 120 from the interior region of the flexible encapsulating member 118. Similarly, a valve or membrane positioned in or adjacent to the second channel 122 may allow a fluid to flow out of the interior region of the flexible encapsulating member 118 while preventing fluid from exiting the second channel 122 and flowing into the interior of the flexible encapsulating member 118. Further, a membrane or cartridge may be positioned in or adjacent to the second channel 122 to assist in degassing fluid within the interior region of the flexible encapsulating member 118. Examples of valves that may be utilized include one-way valves produced by Qosina Corp or Value Plastics. Examples of membranes, such as semipermeable membranes, that may be utilized include those produced by W.L. Gore & Associates, Inc.

In some implementations, during use, a stent 124 may be positioned around the flexible encapsulating member 118 for use in conjunction with lithotripsy. For example, in one method not forming part of the claimed invention , the stent 124 may be placed at the site of a lithotripsy event as a shockwave radiates from the flexible encapsulating member 118 of the EHL probe 100. In another method not forming part of the claimed invention , the stent 124 may be placed at the site of the lithotripsy event shortly after a shockwave radiates from the flexible encapsulating member 118 of the EHL probe 100. Examples of stents 124 that may be used with the EHL probe 100 include the Integrity stent produced by Medtronic or the Promus stent produced by Boston Scientific Corp.

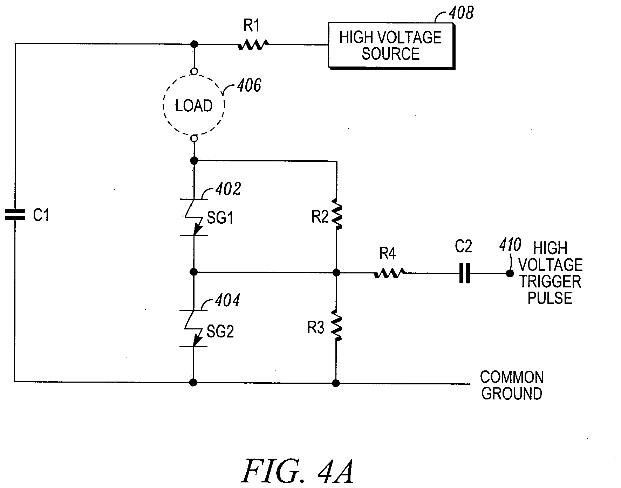

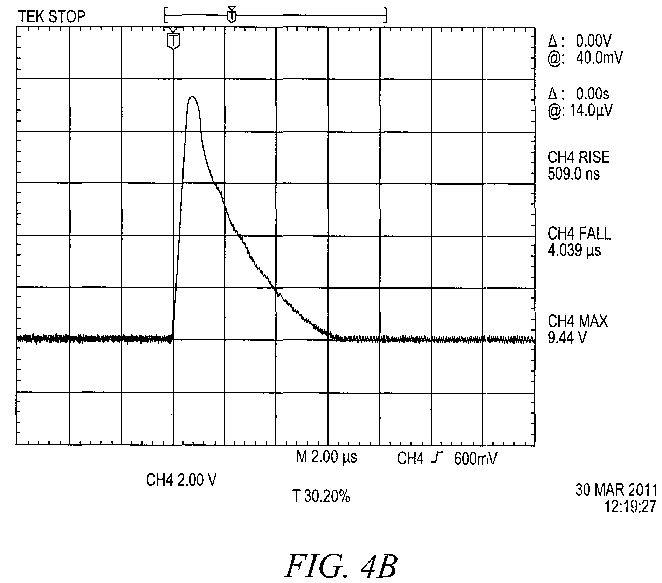

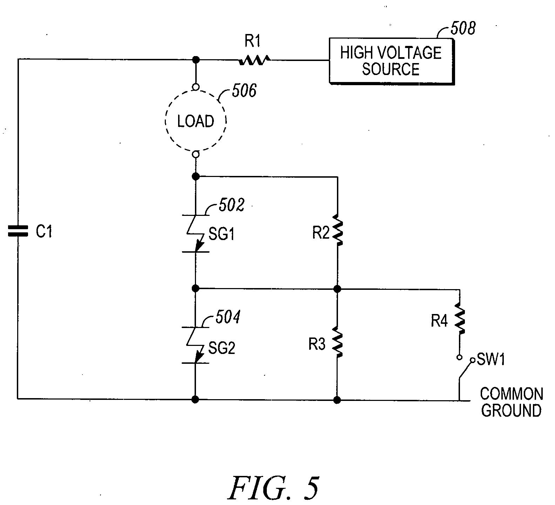

As discussed above, improved power generators that are able to create consistent shockwaves within EHL probes such as those described in conjunction with FIGS. 1-3 are desirable. Electrical sources that utilize multiple spark gaps are described in conjunction with FIGS. 4 and 5 that provide voltage and current to an invasive electrohydraulic lithotripsy probe in a way that result in consistent shockwave generation.

FIG. 4a is a circuit diagram of one implementation of a power generator 400 for an intracorporeal electrohydraulic lithotripter. As discussed in more detail below, the power generator 400 utilizes a first spark gap 402 and a second spark gap 404 for triggering the application of a voltage and current to a load 406 that is connected to terminals of an EHL probe.

In some implementations, the spark gaps 402, 404 are two-electrode devices designed for switching high energy by ionizing gas between two main electrodes. When a high voltage pulse trigger is applied between the electrodes of the spark gap, gas within the spark gap rapidly ionizes between the electrodes and allows current to flow through the spark gap.

Referring to FIG. 4a, during operation, a high voltage source 408 charges capacitor C1 through resister R1. The first and second spark gaps 402, 404 are connected in series with the load 406, which together are positioned in the circuit in parallel with capacitor C1. In some implementations, the first and second spark gaps 402, 404 are selected such that the sum of their breakdown voltages exceeds a maximum voltage to be stored at capacitor C1. Values of resistor R2 and resistor R3 are chosen to proportion the maximum voltage to be stored at capacitor C1 across the first and second spark gaps 402, 404.

To trigger the first and second spark gaps 402, 404, a high voltage pulse is applied at node 410 via resistor R4 and capacitor C2. One example of a resulting high voltage pulse is illustrated in FIG. 4b. The high voltage pulse causes the gas in at least one of the first and second spark gaps 402, 404 to ionize and begin conducting current. When one of the first or second spark gaps 402, 404 begins conducting current, the full potential of capacitor C1 is applied to the other spark gap, which causes the gas in the other spark gap to ionize and begin conducting current. With the first and second spark gaps 402, 404 conducting current, capacitor C1 discharges into the load 406, which is connected to the terminals of an EHL probe, to cause the EHL probe to generate a shockwave as described above.

FIG. 5 is a circuit diagram of another implementation of a power generator 500 for an intracorporeal electrohydraulic lithotripter. Similar to the power generator described above in conjunction with FIG. 4, the power generator 500 utilizes a first spark gap 502 and a second spark gap 504 for triggering the application of a voltage and a current to a load 506 that is connected to terminals of an EHL probe.

During operation a high voltage source 508 charges capacitor C1 through resister R1. The first and second spark gaps 502, 504 are connected in series with the load 506, which together are positioned in the circuit in parallel with capacitor C1. In some implementations the first and second spark gaps 502, 504 are selected such that the sum of their breakdown voltages exceeds the maximum voltage to be stored at capacitor C1. Values of resistor R2 and resistor R3 are chosen to proportion the maximum voltage to be stored at capacitor C1 across the first and second spark gaps 502, 504.

To trigger the first and second spark gaps 502, 504, a switch S1 connected in series with resister R4 across resister R3 is closed. When switch S1 is closed, a voltage drop across the second spark gap 504 decreases, thereby raising the voltage across the first spark gap 502 until the gas within the first spark gap 502 begins to ionize. As the first spark gap 502 conducts current, the full potential of capacitor C1 is applied to the second spark gap 504, which causes the gas in the second spark gap 504 to ionize and begin conducting current. With the first and second spark gaps 502, 504 conducting current, capacitor C1 discharges into the load 406, which is connected to the terminals of an EHL probe, to cause the EHL probe to generate a shockwave as described above.

Switch S1 may be mechanical, electro-mechanical, electronic, ionic vacuum, optoelectronic, or any other type of switch known in the art. Further, in implementations where switch S1 has low leakage current, resisters R2 and R3 may be removed from the circuit.

One concern with EHL probes is a breakdown of the EHL probe, also known as the End of Life of an EHL probe. In some implementations, an electrical source, such as an electrohydraulic generator described above in conjunction with FIGS. 4 and 5, that is connected to an EHL probe may track a number of times it fires an EHL probe. Based on the number of times the EHL probe fires, power levels used during the firing of the EHL probe, and historical End of Life data for EHL probes, the electrical source may then determine when the EHL probe is nearing End of Life.

When an EHL probe nears End of Life, an electrical source may display messages such as "End of Life" or "Change Probe" to warn a doctor to dispose of an EHL probe. In some implementations, an EHL probe may be designed to disable the EHL probe after reaching or nearing End of Life.

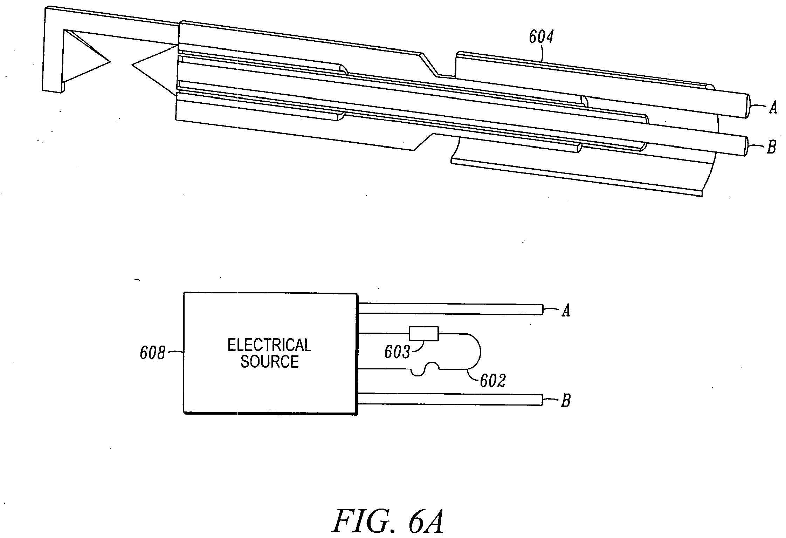

FIG. 6a is a cross-section of an EHL probe 604 including a fusible link 602. Generally, the fusible link 602 is used to disable the EHL probe 604 when the EHL probe nears End of Life. In one implementation, the fusible link 602 may be placed in a connector 606 of the EHL probe 604. However, in other implementations, the fusible link 602 may be placed in other portions of the EHL probe 604.

Generally, an electrical source 608 monitors a number of times the EHL probe 604 fires and power levels used during firing of the EHL probe 604. The electrical source 608 compares the number of times the EHL probe 604 fires and power levels used during firing of the EHL probe 604 to historical End of Life data for EHL probes. When the electrical source 608 determines the EHL probe 604 is near End of Life, the electrical source 608 sends a short blast of high current to the EHL probe 604. In one implementation, the short blast of high current is 150 milliamps. When the short blast of high current reaches the fusible link 602 in the EHL probe 604, the high current causes a fuse within the fusible link 602 to "open" (vaporizing some of the wire to open a circuit), thereby disabling the EHL probe 604. In some implementations, the fusible link 602 includes a sense resistor 603 placed between two pins, however other implementations do not include the sense resistor 603.

In some implementations, the electrical source 608 may measure an interior resistance of an EHL probe 604 so that the electrical source 608 can determine when the EHL probe 604 has been disabled.

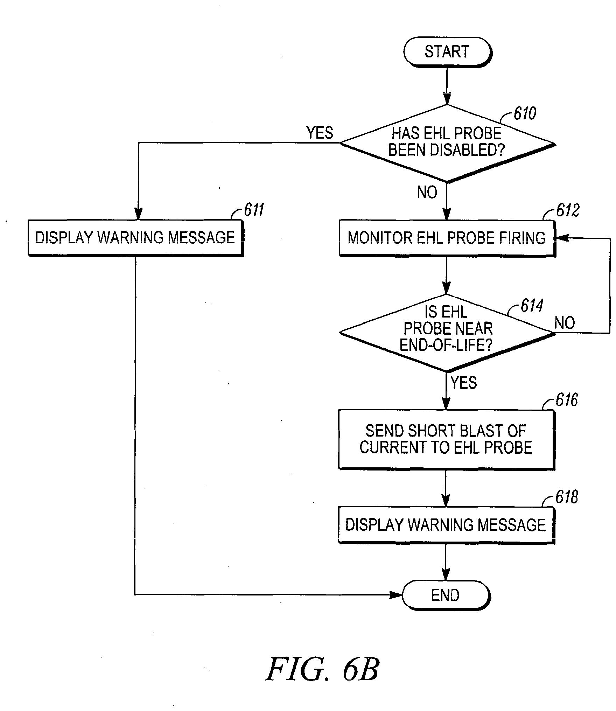

FIG. 6b is a flow chart of a method for using the EHL probe described above with respect to FIG. 6a. At step 610 an electrical source measures an interior resistance of an EHL probe to determine whether the EHL probe has been disabled. The electrical source may perform step 610 when the EHL probe is first connected to the electrical source, periodically, before each firing of the EHL probe, and/or after each firing of the EHL probe.

If the electrical source determines at step 610 that the EHL probe has been disabled, the method proceeds to step 611 where the electrical source displays a message such as "End of Life" or "Change Probe" to warn a doctor to dispose of the EHL probe. However, if the electrical source determines at step 610 that the EHL probe has not been disabled, the method proceeds to step 612 where the electrical source monitors a number of times an EHL probe fires and power levels used during firing of the EHL probe.

At step 614, the electrical source compares the number of times the EHL probe has fired and the power levels used during firing of the EHL probe to historical End of Life data for EHL probes to determine whether the EHL probe is near End of Life. The electrical source may perform step 614 periodically, before each firing of the EHL probe, and/or after each firing of the EHL probe.

If the electrical source determines at step 614 that the EHL probe is not near End of Life, the method loops to step 612 and the electrical source continues to monitor the number of times an EHL probe fires and power levels used during firing of the EHL probe. However, if the electrical source determines at step 612 that the EHL probe is near End of Life, the method proceeds to step 616.

At step 616, the electrical source sends a short blast of high current to the EHL probe to cause a fuse within the fusable link of the EHL probe to open, thereby disabling the EHL probe. At step 618, the electrical source may display a message such as "End of Life" or "Change Probe" to warn a doctor to dispose of the EHL probe.

It will be appreciated that the order of the one or more steps of the method described with respect to FIG. 6b may be changed. Further, in some implementations, the method of FIG. 6b may be implemented in conjunction with a computer-readable storage medium comprising a set of instructions to direct a processor of the electrical source to perform one or more of the above-described acts.

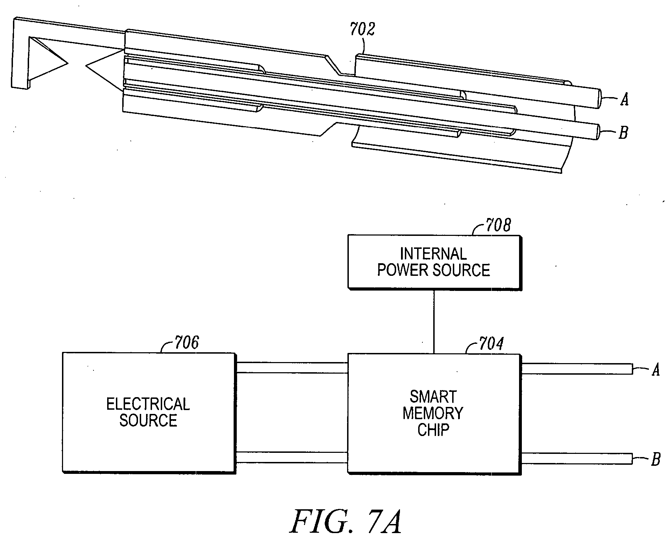

FIG. 7a is a cross-section of an EHL probe 702 including a smart memory chip 704. The smart memory chip 704 may be any flash memory device small enough to be positioned in a connector of the EHL probe 702. One example of such a flash memory device is a 128kx8 monolithic flash chip by White Electronics Designs Corp. (part number WMF-128k8-xclx5). Generally, each time an electrical source 706 fires the EHL probe 702, the electrical source 706 also sends a count signal to the smart memory chip 704. The smart memory chip 704 monitors a number of times the EHL probe 702 has been fired. When the EHL probe 702 is near End of Life, the smart memory chip 704 may disable the EHL probe 702 and prevent the EHL probe 702 from firing.

In some implementations, the EHL probe 702 may include an Internal power source 708 such as a battery so that the smart memory chip 704 may maintain a count of the number of times the EHL probe 702 has fired even when the EHL probe 702 is disconnected from the electrical source 706. In some implementations the smart memory chip 704 may additionally include a unique identifier, such as a serial number, so that the EHL probe 702 is uniquely identified to the electrical source 706 when the EHL probe 702 is first connected to the electrical source 706. The electrical source 706 may use the unique identifier for purposes such as record keeping, performance analysis, or trouble shooting of faulty EHL probes 702.

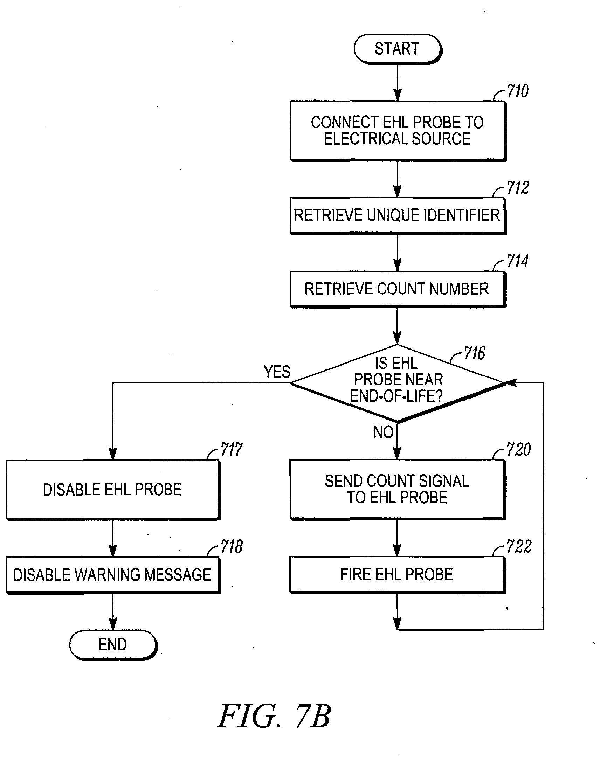

FIG. 7b is a flow chart of a method for using the EHL probe described above with respect to FIG. 7a. At step 710, an EHL probe is connected to the electrical source. At step 712, the electrical source retrieves a unique identifier from a memory of the EHL probe to identify the EHL probe to the electrical source.

At step 714, the electrical source retrieves a count of a number of times the EHL probe has been fired from the memory of the EHL probe. At step 716, the electrical source determines whether the EHL probe is near End of Life by comparing the number of times the EHL probe has been fired to a threshold.

If the electrical source determines at step 716 that the EHL probe is near End of Life, the electrical source disables the EHL probe at step 717. Additionally, the electrical source may display a message such as "End of Life" or "Change Probe" to warn a doctor to dispose of the EHL probe at step 718.

However, if the electrical source determines at step 716 that the EHL probe is not near End of Life, the method proceeds to step 720 where the electrical source sends a count signal to the memory of the EHL probe to increment the number of times the EHL probe has been fired. At step 722, the electrical source fires the EHL probe. The method then loops to step 714.

It will be appreciated that the order of one or more steps of the method described above with respect to FIG. 7b may be changed. For example, the electrical source may fire the EHL probe (step 722) before incrementing the number of times the EHL probe has been fired (step 720). Further, in some implementations, the method of FIG. 7b may be implemented in conjunction with a computer-readable storage medium comprising a set of instructions to direct a processor of the electrical source to perform one or more of the above-described acts.

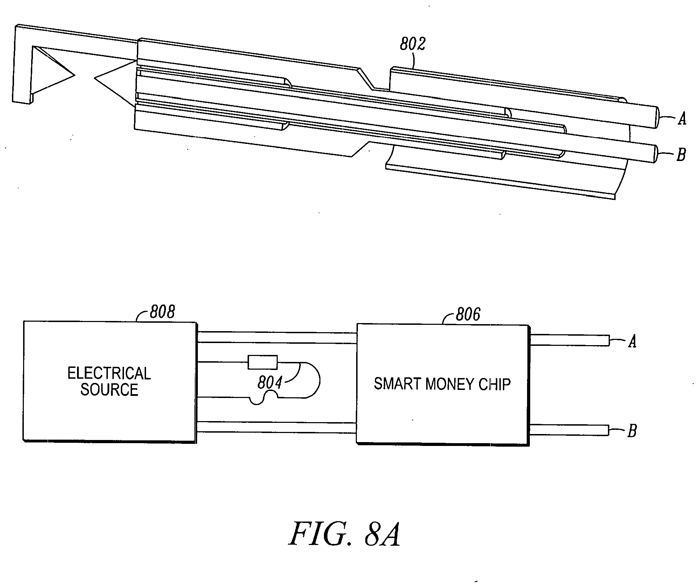

FIG. 8a is a cross-section of an EHL probe 802 including a fusible link 804 and a smart memory chip 806. It will be appreciated that in implementations including both the fusible link 804 and the smart memory chip 806, the fusible link 804 may function as described above in conjunction with FIG. 6 to disable the EHL probe 802 in response to a short blast of high current from an electrical source 808. Additionally, the smart memory chip 806 may function as described above in conjunction with FIG. 7 to disable the EHL probe 802 when the smart memory chip 806 determines based on a number of times the EHL probe 802 has fired that the EHL probe 802 is near End of Life.

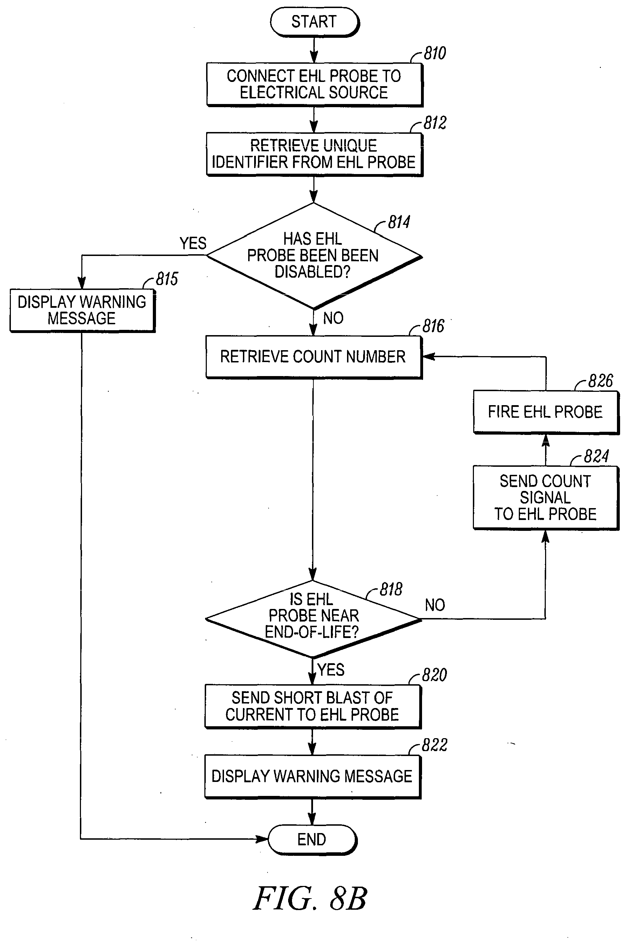

FIG. 8b is a flow chart of a method for using the EHL probe of FIG. 8a. At step 810, an EHL probe is connected to the electrical source. At step 812, the electrical source retrieves a unique identifier from a memory of the EHL probe to identify the EHL probe to the electrical source. At step 814, the electrical source measures an interior resistance of the EHL probe to determine whether the EHL probe has been disabled.

If the electrical source determines at step 814 that the EHL probe has been disabled, the method proceeds to step 815 where the electrical source displays a message such as "End of Life" or "Change Probe" to warn a doctor to dispose of the EHL probe. However, if the electrical source determines at step 814 that the EHL probe has not been disabled, the method proceeds to step 816.

At step 816, the electrical source retrieves a count of a number of times the EHL probe has been fired from the memory of the EHL probe. At step 818, the electrical source determines whether the EHL probe is near End of Life by comparing the number of times the EHL probe has been fired to a threshold.

If the electrical source determines at step 818 that the EHL probe is near End of Life, the method proceeds to step 820 where the electrical source sends a short blast of high current to the EHL probe to cause a fuse within the fusable link of the EHL probe to open, thereby disabling the EHL probe. At step 822, the electrical source may display a message such as "End of Life" or "Change Probe" to warn a doctor to dispose of the EHL probe.

If the electrical source determines at step 818 that the EHL probe is not near End of Life, the method proceeds to step 824 where the electrical source sends a count signal to the memory of the EHL probe to increment the number of times the EHL probe has been fired. At step 826, the electrical source fires the EHL probe. The method then loops to step 816.

It will be appreciated that the order of one or more steps of the method described above with respect to FIG. 8b may be changed. Further, in some implementations, the method of FIG. 8b may be implemented in conjunction with a computer-readable storage medium comprising a set of instructions to direct a processor of the electrical source to perform one or more of the above-described acts.

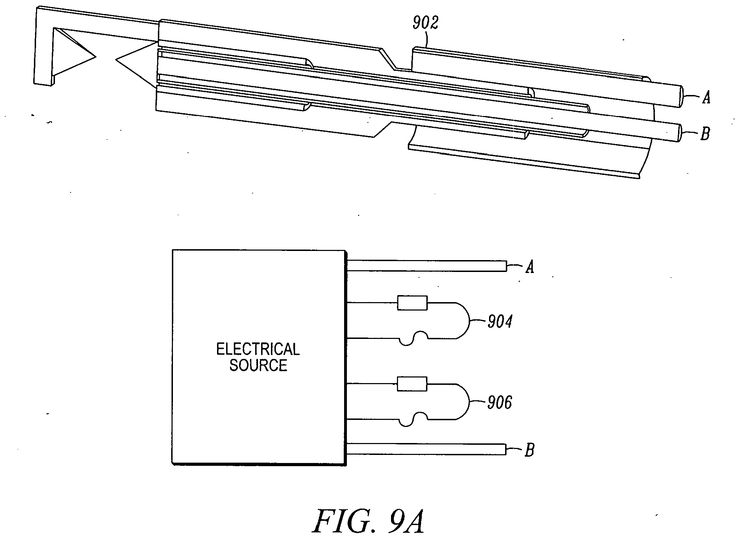

FIG. 9a is a cross-section of an EHL probe 902 including a first fusible link 904 and a second fusible link 906. Generally, when the EHL probe 902 is first fired, the first fusible link 904 is blown so that the EHL probe cannot be reused (protecting its single use designation). Additionally, when an electrical source determines the EHL probe 902 is near End of Life, the electrical source may send a short blast of high current to the EHL probe 902 to blow the second fusible link 906 as described above in conjunction with FIG. 6, thereby disabling the EHL probe 902.

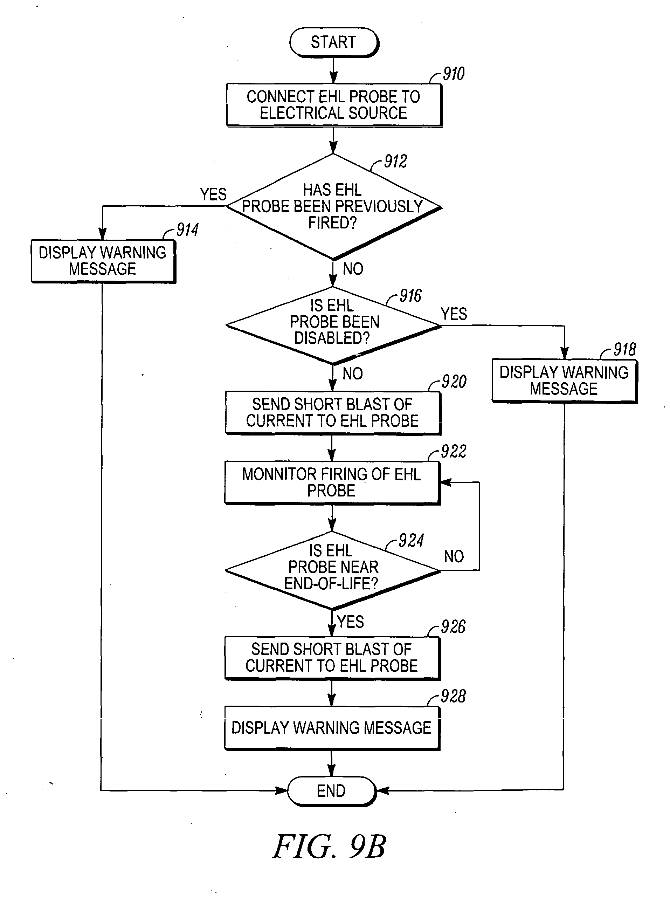

FIG. 9b is a flow chart of a method for using the EHL probe described above with respect to FIG. 9a. At step 910 an EHL probe is connected to an electrical source. At step 912, the electrical source measures an interior resistance of the EHL probe with respect to a first fuse to determine if the EHL probe has been previously fired. If the electrical source determines at step 912 that the EHL probe has been fired, the method proceeds to step 914 where the electrical source displays a message such as "End of Life" or "Change Probe" to warn a doctor to dispose of the EHL probe.

If the electrical source determines at step 912 that the EHL probe has not been previously fired, the method proceeds to step 916 where the electrical source measures an interior resistance of the EHL probe with respect to a second fuse to determine whether the EHL has been disabled because it is near End of Life. If the electrical source determines at step 916 that the EHL probe has been disabled, the method proceeds to step 918 where the electrical source displays a message such as "End of Life" or "Change Probe" to warn a doctor to dispose of the EHL probe.

If the electrical source determines at step 918 that the EHL probe has not been disabled, the method proceeds to step 920 where the electrical source sends a short blast of high current to the EHL probe to cause the first fuse within the fuseable link of the EHL probe to open before the EHL probe is fired for the first time. At step 922 the electrical source monitors a number of times an EHL probe fires and power levels used during firing of the EHL probe.

At step 924, the electrical source compares the number of times the EHL probe has fired and the power levels used during firing of the EHL probe to historical End of Life data for EHL probes to determine whether the EHL probe is near End of Life. If the electrical source determines at step 924 that the EHL probe is not near End of Life, the method loops to step 922 and the electrical source continues to monitor the number of times an EHL probe fires and power levels used during firing of the EHL probe.

If the electrical source determines at step 924 that the EHL probe is near End of Life, the method proceeds to step 926 where the electrical source sends a short blast of high current to the EHL probe to cause the second fuse within the fusable link of the EHL probe to open, thereby disabling the EHL probe. At step 928, the electrical source may display a message such as "End of Life" or "Change Probe" to warn a doctor to dispose of the EHL probe.

It will be appreciated that the order one or more steps of the method described with respect to FIG. 9b may be changed. Further, in some implementations, the method of FIG. 9b may be implemented in conjunction with a computer-readable storage medium comprising a set of instructions to direct a processor of the electrical source to perform one or more of the above-described acts.

It is intended that the foregoing detailed description be regarded as illustrative rather than limiting, and that it be understood that it is the following claims that are intended to define the scope of this invention.

## Claims

1. An invasive lithotripter probe system comprising:
an invasive lithotripter tip (101) for use with a lithotripter probe (100), the lithotripter tip (101) comprising:
a first conic electrode (104) positioned at a distal end (108) of the lithotripter tip; and
a second conic electrode (106), an end of which is coaxially aligned with an end of the first conic electrode; and
an electrical source (400, 500) configured to charge the first conic electrode (104) to a first polarity, the electrical source comprising a two-electrode spark gap triggering system;
wherein the first (104) and second conic electrodes (106) are positioned on the lithotripter tip (101) such that an electric arc between the ends of the first and second conic electrodes causes a shockwave that is toroid shaped to radiate from the lithotripter tip (101) ,
wherein the two-electrode spark gap triggering system comprises:
a first spark gap (402, 502);
a second spark gap (404, 504) electrically connected in series with the first spark gap; and
a storage capacitor (C1) electrically connected in parallel with the first (402, 502) and second spark gaps (404, 504);
wherein the first conic electrode (104) is electrically connected to the storage capacitor (C1) via the serially connected first and second spark gaps; and
wherein when the first and second spark gaps conduct, the storage capacitor is configured to discharge to the first conic electrode, thereby causing the electric arc between the ends of the first and second conic electrodes
wherein the lithotripter tip is at least partially surrounded by a flexible encapsulating member (118) , and
wherein the flexible encapsulating member (118) defines a channel (120) configured to receive a liquid for insertion into the flexible encapsulating member, and
comprising a one-way valve configured to prevent the liquid from being extracted from the flexible encapsulating member (118) through the channel (120).

2. The invasive lithotripter probe system of claim 1,
wherein a high voltage trigger pulse applied to a common node between the first spark gap and the second spark gap results in the first and second spark gaps conducting, and the storage capacitor discharging to the first conic electrode.

3. The invasive lithotripter probe system of claim 1,
wherein causing a voltage drop at a common node between the first spark gap and the second spark gap results in the first and second spark gaps conducting, and the storage capacitor discharging to the first conic electrode.

4. The invasive lithotripter probe system of claim 1,
wherein the flexible encapsulating member (118) defines a channel (122) configured to receive a liquid from the flexible encapsulating member for extraction from the flexible encapsulating member.

5. The invasive lithotripter probe system of claim 4,
wherein the channel (122) comprises a one-way valve configured to prevent the liquid from being inserted into the flexible encapsulating member through the channel.

6. The invasive lithotripter probe system of claim 1,
wherein the flexible encapsulating member (118) defines a channel (122) configured for extracting gas from a fluid within an interior of the flexible encapsulating member.

7. The invasive lithotripter probe system of claim 1, further comprising a stent (124) positioned around the flexible encapsulating member (118) .

## Patentansprüche

1. Invasives Lithotriptersonden-System, umfassend:
eine invasive Lithotripterspitze (101) zur Verwendung mit einer Lithotriptersonde (100), wobei die Lithotripterspitze (101) umfasst:
eine erste konische Elektrode (104), die an einem distalen Ende (108) der Lithotripterspitze angeordnet ist; und
eine zweite konische Elektrode (106), deren eines Ende koaxial mit einem Ende der ersten konischen Elektrode ausgerichtet ist; und
eine elektrische Quelle (400, 500), die so konfiguriert ist, dass sie die erste konische Elektrode (104) auf eine erste Polarität auflädt, wobei die elektrische Quelle ein Zwei-Elektroden-Funkenstrecken-Auslösesystem umfasst;
wobei die erste (104) und die zweite konische Elektrode (106) auf der Lithotripterspitze (101) so positioniert sind, dass ein elektrischer Lichtbogen zwischen den Enden der ersten und der zweiten konischen Elektrode eine Stoßwelle verursacht, die toroidförmig ist und von der Lithotripterspitze (101) ausstrahlt,
wobei das Zwei-Elektroden-Funkenstrecken-Auslösesystem umfasst:
eine erste Funkenstrecke (402, 502);
eine zweite Funkenstrecke (404, 504), die mit der ersten Funkenstrecke (402, 502) elektrisch in Reihe geschaltet ist; und
einen Speicherkondensator (C1), der mit der ersten (402, 502) und der zweiten Funkenstrecke (404, 504) elektrisch parallelgeschaltet ist;
wobei die erste konische Elektrode (104) über die in Reihe geschalteten ersten und zweiten Funkenstrecken elektrisch mit dem Speicherkondensator (C1) verbunden ist; und
wobei der Speicherkondensator so konfiguriert ist, dass er sich auf die erste konische Elektrode entlädt, wenn die erste und die zweite Funkenstrecke leiten, wodurch der Lichtbogen zwischen den Enden der ersten und der zweiten konischen Elektrode entsteht,
wobei die Lithotripterspitze zumindest teilweise von einem flexiblen Einkapselungselement (118) umgeben ist, und
wobei das flexible Einkapselungselement (118) einen Kanal (120) definiert, der so konfiguriert ist, dass er eine Flüssigkeit zum Einführen in das flexible Einkapselungselement aufnimmt, und
ein Einwegventil umfasst, das so konfiguriert ist, dass es verhindert, dass die Flüssigkeit aus dem flexiblen Einkapselungselement (118) durch den Kanal (120) herausgezogen wird.

2. Invasives Lithotriptersonden-System nach Anspruch 1, wobei ein an einem gemeinsamen Knoten zwischen der ersten Funkenstrecke und der zweiten Funkenstrecke angelegter Hochspannungsauslöseimpuls dazu führt, dass die erste und die zweite Funkenstrecke leitend werden und sich der Speicherkondensator auf die erste konische Elektrode entlädt.

3. Invasives Lithotriptersonden-System nach Anspruch 1, wobei das Verursachen eines Spannungsabfalls an einem gemeinsamen Knoten zwischen der ersten Funkenstrecke und der zweiten Funkenstrecke dazu führt, dass die erste und die zweite Funkenstrecke leitend werden und der Speicherkondensator sich an die erste konische Elektrode entlädt.

4. Invasives Lithotriptersonden-System nach Anspruch 1, wobei das flexible Einkapselungselement (118) einen Kanal (122) definiert, der so konfiguriert ist, dass er eine Flüssigkeit aus dem flexiblen Einkapselungselement zur Extraktion aus dem flexiblen Einkapselungselement aufnimmt.

5. Invasives Lithotriptersonden-System nach Anspruch 4, wobei der Kanal (122) ein Einwegventil umfasst, das so konfiguriert ist, dass es verhindert, dass die Flüssigkeit durch den Kanal in das flexible Einkapselungselement eingeführt wird.

6. Invasives Lithotriptersonden-System nach Anspruch 1, wobei das flexible Einkapselungselement (118) einen Kanal (122) definiert, der zum Extrahieren von Gas aus einem Fluid im Inneren des flexiblen Einkapselungselements konfiguriert ist.

7. Invasives Lithotriptersonden-System nach Anspruch 1, das ferner einen Stent (124) umfasst, der um das flexible Einkapselungselement (118) herum angeordnet ist.

## Revendications

1. Système invasif sonde lithotripteur comprenant :
une pointe de lithotripteur (101) invasive pour l'utilisation avec une sonde lithotripteur (100), la pointe de lithotripteur (101) comprenant :
une première électrode conique (104) positionnée au niveau d'une extrémité distale (108) de la pointe de lithotripteur ; et
une seconde électrode conique (106), dont une extrémité est alignée de manière coaxiale sur une extrémité de la première électrode conique ; et
une source d'électricité (400, 500) conçue pour charger la première électrode conique (104) à une première polarité, la source d'électricité comprenant un système de déclenchement à écartement d'électrodes à deux électrodes ;
la première (104) et la seconde électrode conique (106) étant positionnées sur la pointe de lithotripteur (101) de sorte qu'un arc électrique entre les extrémités de la première et de la seconde électrode conique entraîne une onde de choc qui est de forme toroïdale pour irradier depuis la pointe de lithotripteur (101),
le système de déclenchement à écartement d'électrodes à deux électrodes comprenant :
un premier écartement d'électrodes (402, 502) ;
un second écartement d'électrodes (404, 504) électriquement connecté en série avec le premier écartement d'électrodes ; et
un condensateur de stockage (C1) électriquement connecté en parallèle avec le premier (402, 502) et le second écartement d'électrodes (404, 504) ;
la première électrode conique (104) étant électriquement connectée au condensateur de stockage (C1) par l'intermédiaire du premier et du second écartement d'électrodes connectés en série ; et
lorsque le premier et le second écartement d'électrodes effectuent la conduction, le condensateur de stockage est conçu pour effectuer une décharge au niveau de la première électrode conique, causant ainsi l'arc électrique entre les extrémités de la première et de la seconde électrode conique ;
la pointe de lithotripteur étant au moins partiellement entourée d'un élément d'encapsulation souple (118), et
l'élément d'encapsulation souple (118) définissant un canal (120) conçu pour recevoir un liquide pour l'insertion dans l'élément d'encapsulation souple, et
comprenant une vanne à une voie conçue pour empêcher le liquide d'être extrait de l'élément d'encapsulation souple (118) à travers le canal (120).

2. Système invasif sonde lithotripteur selon la revendication 1,
une impulsion de déclenchement à haute tension appliquée à un nœud commun entre le premier écartement d'électrodes et le second écartement d'électrodes résultant en le premier et le second écartement d'électrodes effectuant la conduction, et le condensateur de stockage effectuant une décharge au niveau de la première électrode conique.

3. Système invasif sonde lithotripteur selon la revendication 1,
le fait d'amener une chute de tension au niveau d'un nœud commun entre le premier écartement d'électrodes et le second écartement d'électrodes résultant en le premier et le second écartement d'électrodes effectuant la conduction, et le condensateur de stockage effectuant une décharge au niveau de la première électrode conique.

4. Système invasif sonde lithotripteur selon la revendication 1,
l'élément d'encapsulation souple (118) définissant un canal (122) conçu pour recevoir un liquide depuis l'élément d'encapsulation souple pour l'extraction depuis l'élément d'encapsulation souple.

5. Système invasif sonde lithotripteur selon la revendication 4,
le canal (122) comprenant une vanne à une voie conçue pour empêcher le liquide d'être inséré dans l'élément d'encapsulation souple à travers le canal.

6. Système invasif sonde lithotripteur selon la revendication 1,
l'élément d'encapsulation souple (118) définissant un canal (122) conçu pour extraire du gaz d'un fluide depuis un intérieur de l'élément d'encapsulation souple.

7. Système invasif sonde lithotripteur selon la revendication 1, comprenant en outre une endoprothèse (124) positionnée autour de l'élément d'encapsulation souple (118).
